# EUROPEAN PATENT APPLICATION

(11) **EP 4 063 384 A1**
(43) Date of publication of application: **28.09.2022**
(21) Application number: 21164306.9
(22) Date of filing: 23.03.2021
(51) Int. Cl.: C07K 16/10, A61P 31/14

(54) **HUMAN NEUTRALIZING ANTIGEN SPECIFIC PROTEINS FOR SPIKE-RBD OF SARS-COV-2**

(71) Applicant: Ceinge Biotecnologie Avanzate SCarl, 80145 Napoli (IT)
(72) Inventor: DE LORENZO, Claudia, 80122 NAPOLI (IT); ZAMBRANO, Nicola, 80020 CASAVATORE (NA) (IT); ZOLLO, Massimo, 80131 NAPOLI (IT)
(74) Representative: Bianchetti & Minoja SRL

(57) **Abstract**

The invention concerns antigen specific proteins, in particular antibodies of fragments thereof, specifically binding to RBD domain and interfering in the interaction of Spike protein with ACE2 receptor, said antigen specific proteins having neutralizing activity for SARS-CoV2 virus infection.

## Description

The present invention concerns antigen specific proteins, in particular antibodies of fragments thereof, specifically binding to RBD domain and interfering in the interaction of Spike protein with ACE2 receptor, said antigen specific proteins having neutralizing activity for SARS-CoV2 virus infection.

### Background of the invention

The novel coronavirus SARS-CoV-2, responsible for the Severe Acute Respiratory Syndrome known as Covid-19, infects epithelial cells of the respiratory tract, causing typical signs such as fever, dry cough, fatigue and dyspnea^{1,2}. A small fraction of patients however progresses towards a severe form of pneumonia requiring the hospitalization or in some cases intensive care unit treatments³. The outbreaks of the new pandemic at the end of 2019 have bolstered the research of new vaccines and biomedical treatments urgently needed to prevent or reduce symptoms associated to the new SARS-CoV-2 coronavirus infection⁴.

Coronaviruses are large enveloped viruses, endowed with a positive sense RNA genome, belonging to the family of coronaviridae. Up to date, they were considered pathogens with a veterinary relevance as they were used to infect mammalian and avian species with a limited impact on human beings⁵. Since the beginning of 21^{st} century coronaviruses have crossed the barrier between species, leading to Severe Acute Respiratory Syndrome Coronavirus (SARS-CoV) in 2003 and Middle-East Respiratory Syndrome Coronavirus (MERS-CoV) in 2012⁶.

Cell entry of SARS-CoV-2 Coronavirus is mediated by the Spike glycoprotein that is present in multiple copies creating an extensive crown on virus envelope. Spike glycoprotein is a class 1 fusion protein produced as a single polypeptide of about 1300 amino acids that trimerizes upon folding⁷. It comprises two functional subunits called S1 and S2: S1 represents the apex of the trimer and by its receptor binding domain (RBD), including residues from 331 to 524 of the S protein, promotes the attachment and fusion of virus particles with host membranes; S2 subunit, anchored to the viral membrane, contains a hydrophobic fusion loop and two heptad repeat regions (HR1 and HR2)⁸. The interaction of Spike with the host receptor Angiotensin-Converting Enzyme 2 (ACE2) represents the first step of the infection, followed by proteolysis and conformational changes of Spike translating into the exposure of the fusion loop and its insertion into the target cell membrane with the release of viral genome into the cells⁹⁻¹¹. Consequently, the RBD of S1 subunit is critical for determining host specificity and cell tropism^{12,13}. The structure of the SARS-CoV-2 spike receptor-binding domain bound to the ACE2 receptor has been disclosed by Lan, J. et al.²²

To date, many progresses have been done in the scientific approaches against SARS-CoV-2. These include the inhibition of virus entry, fusion or replication, as well as the suppression of excessive inflammatory responses with different drugs such as chloroquine, remdesivir and dexamethasone, respectively^{14,15}. Furthermore, three vaccines have been recently approved by FDA for preventative use and several other candidates are being tested by ongoing phase III clinical trials^{16-19,27}.

Among these approaches, monoclonal antibodies (mAbs) targeting the Spike glycoprotein represent good candidates to interfere in the Spike/ACE2 interaction, preventing virus cell entry. Therefore, the development of anti-Spike mAbs provides a weapon to contain the spreading of the virus, in order to prevent the worsening of the symptoms, especially in high risk patients. Until now, the U.S. Food and Drug Administration (FDA) issued an emergency use authorization (EUA) for the mAb bamlanivimab (by Eli Lilly and Company's) for the treatment of mild-to-moderate SARS-CoV-2 adult and pediatric patients²⁰.

Since anti-spike mAbs, used individually, might be unable to block the virus entry in the case of resistant mutations in the virus, it would be advantageous to generate cocktails of mAbs. To this aim, we designed an innovative strategy for the isolation of novel human anti-Spike-RBD mAbs, by using the phage display technology. The selection was based on two different parallel approaches that allowed for the isolation of a number of antibodies endowed with the ability to bind with high specificity to the RBD domain of Spike protein responsible for the infectivity of the virus¹¹ in order to interfere in the binding of Spike RBD to the ACE2 receptor, and to neutralize the virus before its entry into the cells. Recently, novel human antibodies raised against SARS-CoV-2 spike protein S1 and S2 domains or against SARS-CoV-2 nucleoprotein were obtained and generated as fully human IgG1 antibodies^{21, 25, 28, 29}. Cross-reactive patterns between SARS-CoV-2 proteins and autoimmune target proteins were found to play a role in the systemic inflammatory response in patients affected by Covid-19 leading to the development of autoimmune diseases post-infection in susceptible subgroups of patients³⁰.

### Description of the invention

The present invention refers to antigen specific proteins which specifically bind to RBD domain in a low nanomolar range and they are able to interfere in the interaction of Spike protein with ACE2 receptor, either used as purified protein or when expressed on cells in its native conformation. Furthermore, said antigen specific proteins have shown neutralizing activity against SARS-CoV2 virus infection in cell cultures.

The antigen specific protein binding to SARS-CoV2 Spike RBD of the invention comprises:
i. a heavy chain variable region comprising an amino acid sequence selected from SEQ ID NO: 1, SEQ ID NO: 3 and SEQ ID NO: 4 and
ii. a light chain variable region comprising the amino acid sequence SEQ ID NO: 2.

The antigen specific protein may be in form of a human antibody, a humanized antibody, a chimeric antibody, a monoclonal antibody, a multi-specific antibody, a recombinant antibody, an antigen-binding antibody fragment, a single chain antibody, a single chain variable fragment antibody, a diabody, an antibody-like protein or a fragment thereof, a Fab fragment, an F(ab)2 fragment, an antibody mimetic, an IgG1 antibody, an IgG2 antibody, an IgG3 antibody or an IgG4 antibody, specifically an IgG4 antibody.

The invention also provides a method for the preparation of the antigen specific proteins, comprising the step of isolating anti-Spike scFv-phages specific for RBD by a first panning phages on human Spike RBD-Fc recombinant target protein followed by a selective elution with an excess of receptor ACE2-Fc chimeric protein which binds to Spike RBD.

The invention also provides nucleotide sequences coding for the light and heavy chain variable regions of said antigen specific proteins. SEQ ID NO 5 is the DNA sequence coding for the heavy chain variable region having SEQ ID NO 1; SEQ ID NO 6 is the DNA sequence coding for the light chain variable region having SEQ ID NO 2; SEQ ID NO 7 is the DNA sequence coding for the heavy chain variable region having SEQ ID NO 3; SEQ ID NO 8 is the DNA sequence coding for the heavy chain variable region having SEQ ID NO 4.

The antigen specific proteins of the invention and the corresponding nucleotide sequences are useful for therapeutic and diagnostic purposes. For therapeutic purposes, the antigen specific proteins may be formulated with suitable carrier or excipient to provide pharmaceutical compositions which may be administered according to known methods and regimens, as conventionally used for antibodies or fragments thereof. The corresponding nucleotide sequences may also be used as active ingredients of compositions useful for the therapy or prevention of the Sars-CoV-2 infection.

Fully human anti-SARS-CoV-2 scFvs and neutralizing full length antibodies targeting distinct epitopes on the spike RBD prepared according to the invention can be also combined to improve treatment efficacy and to reduce the risk of treatment-escape of viral variants.³²

The antigen specific proteins of the invention may also be used to set up a detection method to reveal the level of Spike protein in biological samples, useful for diagnosis and monitoring of the viral disease.To this purpose, the sample is incubated with the antigen specific protein and the binding of the antigen to the antigen specific protein is measured according to standard methods, i.e. by measuring an output generated by a suitable label (an enzyme, a fluorophore or chromophore) present on the diagnostic agent. An ELISA method may be conveniently used.

### Description of the Figures

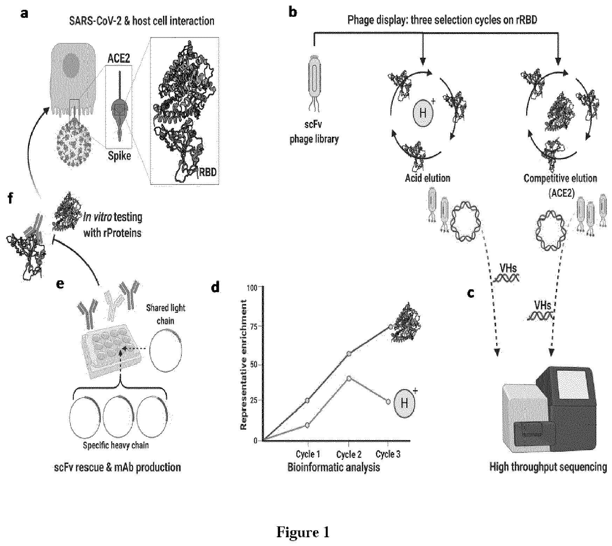

**Figure 1****: Schematic representation of selection and screening strategies for identification of SARS-CoV-2 neutralizing mAbs. a** Cartoon representing SARS-CoV-2 host cell attachment mediated via ACE2-Spike interaction. The ACE2-RBD interaction in the box is adapted from PDB 6M0J²². **b** Phage display of scFv library was carried out on recombinant SARS-CoV-2 RBD-Fc protein; acidic or competitive elution were implemented. **c** Variable heavy chains of scFvs were extracted from sub-libraries and sequenced by MiSeq Illumina. **d** The trend of enrichment of given clones was evaluated within and between the selection cycles. **e** Potential binders (scFvs) were converted into fully human IgG4 mAbs in a high yield expressing cell line (HEK293ES_1). **f** Binding to rRBD and competition with rACE2 were evaluated in vitro with recombinant protein assay. Neutralization capacity of RBD-specific mAbs was further evaluated for blocking SARS-CoV-2 replication.

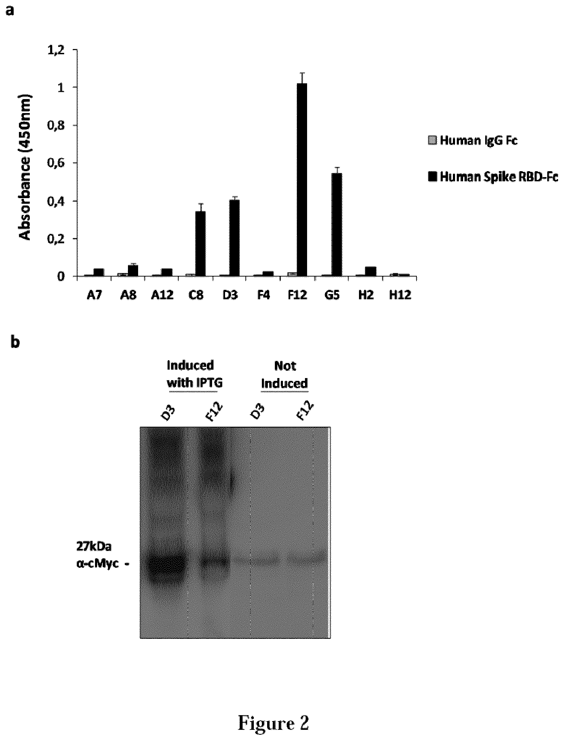

**Figure 2****: Screening and expression of anti-Spike scFvs. a** screening of positive phage clones by ELISA assay on human Spike RBD-Fc recombinant protein (black bars) or human IgGFc used as a negative control in parallel assays (grey bars). **b** Western blotting analysis with the anti-c-myc antibody of the periplasmic extracts of the cells transformed with the two selected positive clones, D3 and F12, expressed in the absence or in the presence of IPTG, used for induction.

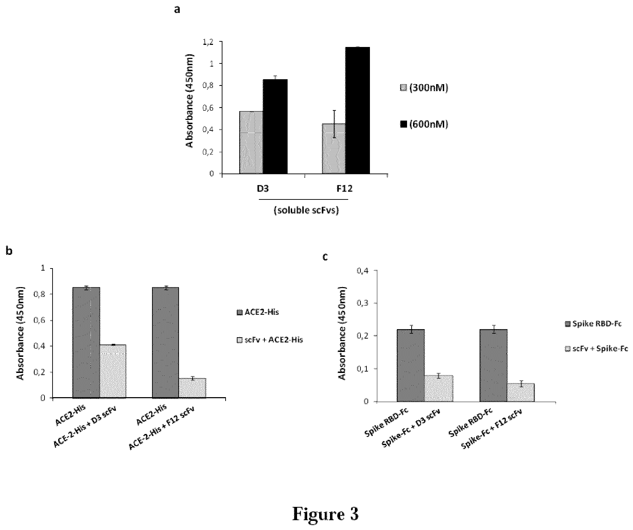

**Figure 3** **Binding of scFvs to Spike RBD and their competition with ACE2. a** ELISA assay to test the binding of D3 and F12 as soluble scFvs on immobilized Spike RBD-Fc recombinant protein by using the concentration of 300 nM (grey bars) or 600 nM (black bars). **b** The binding of ACE2-His to immobilized Spike RBD protein in the absence (dark grey bars) or in the presence (light grey bars) of the indicated soluble scFvs. **c** Competitive ELISA assay was performed by measuring the binding of Spike RBD-Fc protein on ACE2-positive VERO E6 cells in the absence or in the presence of D3 and F12 scFvs.

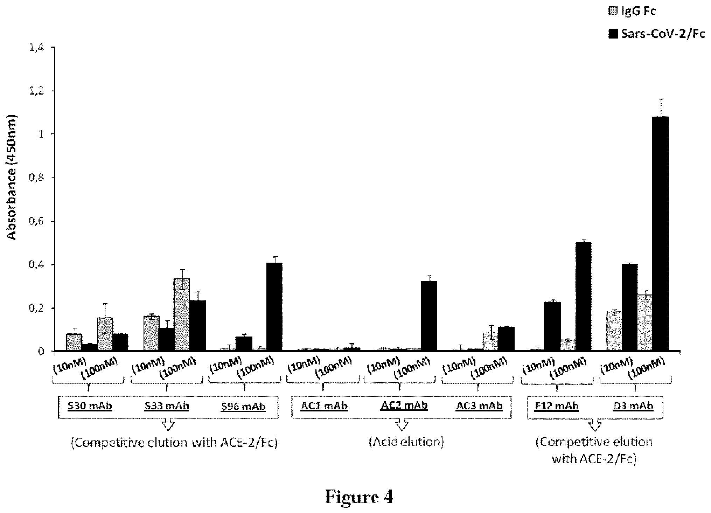

**Figure 4****: ELISA assay on human Spike RBD of the converted mAbs.** The eight converted mAbs were tested on human Spike RBD-Fc recombinant protein (black bars) or on human Fc (grey bars), used as negative control in parallel assays.

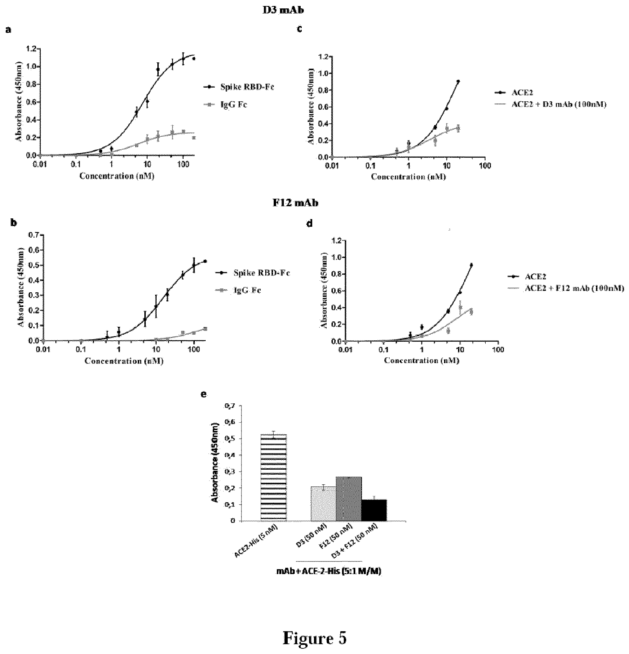

**Figure 5****: Binding affinity of D3 and F12 mAbs for Spike and their competition with ACE-2. a-c** ELISA assays were performed by testing D3 (**a**) and F12 (**c**) mAbs at increasing concentrations (0.5-200 nM) on human Spike RBD-Fc chimeric protein (black curves). In parallel the Fc domain (grey curves) was used as negative control.
   **b-d** Competitive ELISA assays were performed by measuring the binding of ACE2-His protein to Spike RBD in the absence or in the presence of D3 (**b**) and F12 (**d**) mAb used at a concentration of 100 nM.
   **e** Competitive ELISA assays were performed by measuring the binding of ACE2-His to RBD in the absence (striped bar) or in the presence of the indicated mAbs used alone (gray bars) or in combination (black bars) at a concentration of 100 nM.
   The binding values were reported as the mean of at least three determinations obtained in three independent experiments. Error bars depicted means ± SD.

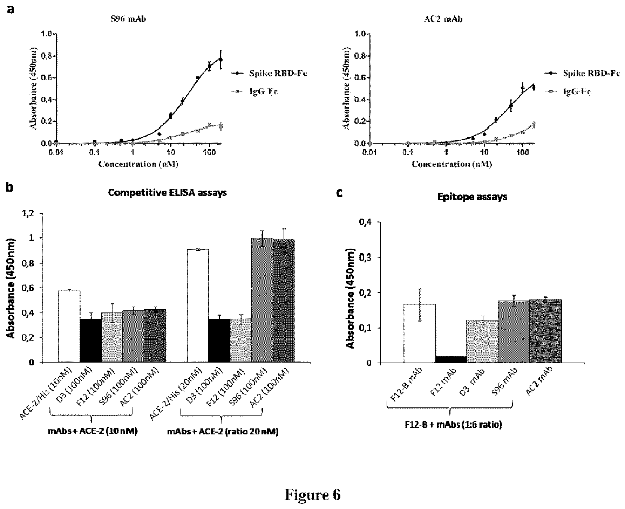

**Figure 6****: ELISA assays on Spike RBD to test the binding affinity of S96 and AC2 mAbs and their competition with ACE-2. a** The binding affinity and specificity for Spike RBD of S96 and AC2 mAbs was evaluated by testing each mAb at increasing concentrations (0.5-200 nM) on Spike RBD-Fc chimeric protein or Fc, used as a negative control.
   **b** Competitive ELISA assays were performed by measuring the binding of ACE2-His to RBD in the absence (white bars) or in the presence of the indicated mAbs (gray and black bars) at a concentration of 100 nM.
   **c** Competitive ELISA assays to determine the epitope binning were performed by measuring the binding of Biotinylated F12 (F12-B) mAb to RBD, pre-incubated in the absence (white bars) or in the presence of the indicated mAbs (grey and black bars). The binding values were reported as the mean of at least three determinations obtained in three independent experiments. Error bars depicted means ± SD.

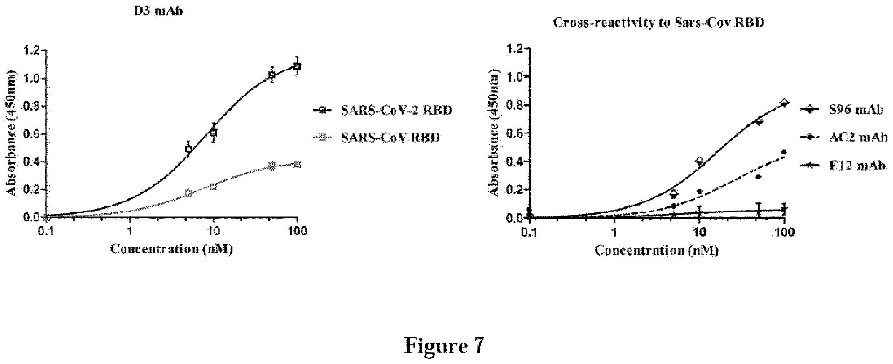

**Figure 7****: Cross-reactivity of the novel mAbs for SARS-CoV RBD.** The binding of D3, S96, F12 or AC2 mAb, tested at increasing concentrations (5-100 nM), on SARS-CoV RBD protein was analyzed in comparison with the binding of D3 to SARS-CoV-2 RBD protein (squares, black curve) by ELISA. The binding values were reported as the mean of at least three determinations obtained in three independent experiments. Error bars depicted means ± SD.

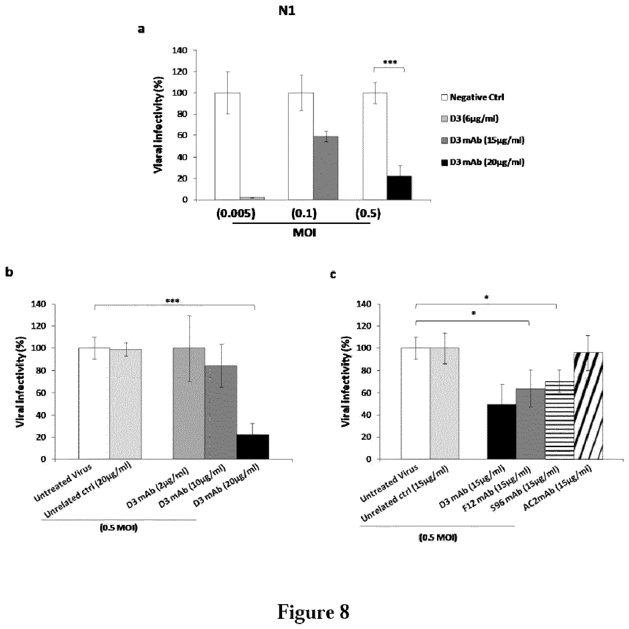

**Figure 8****: Neutralization assays to test the biological effects of anti-Spike mAbs.** VERO E6 cells were infected with the SARS-CoV-2 virus at the indicated MOI, preincubated in the absence or presence of each mAb used at the indicated concentrations. **a** Viral infectivity was measured by RT-PCR of the cell extracts after washes by analyzing the expression of N1 viral gene. The values are expressed as percentage with respect to the negative untreated control. **b** Dose dependent effects of D3 mAb on viral infectivity were measured at the highest viral load. **c** Comparison of the efficiency of the different mAbs for inhibiting virus infectivity of cell cultures at a concentration of 15µg/ml.

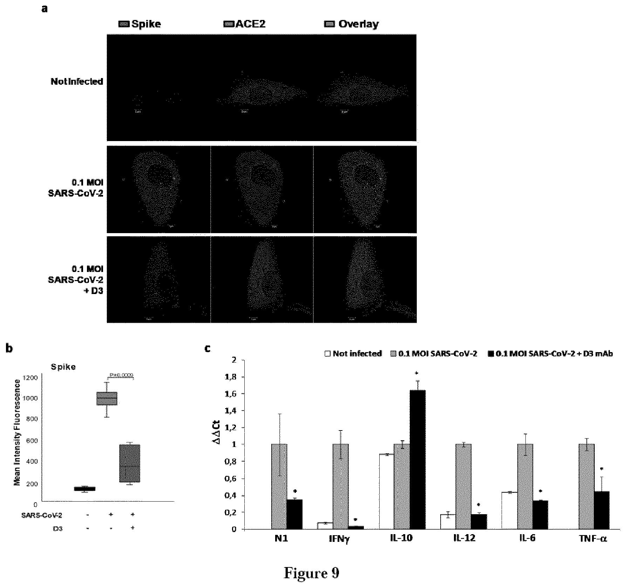

**Figure 9****: Analysis of human primary cells infected with the UK variant in the absence or presence of D3, by immunofluorescence assays and RT-PCR. a** The cells untreated or infected with the UK variant of SARS-CoV2, in the absence or presence of D3 for 72 hours at 37°C, were fixed, washed and permeabilised, as described in the Methods. After blocking, the slides were incubated with the relevant primary antibodies overnight at 4 °C: anti-ACE2 (1:50; ab15348; Abcam) or anti-SARS Spike (1:50; ab272420; Abcam), followed by the relevant secondary anti-mouse Alexa Fluor 488 (1:100; ab150113; Abcam) and anti-Rabbit Alexa Fluor 647 (1:100; ab150075; Abcam), respectively. DNA was stained with DAPI (1:5000; #62254; Thermo Fisher). Microscopy images were obtained with the Elyra 7 platform (Zeiss) with the optical Lattice SIM technology, using the 63 × oil immersion objective. **b** Quantification of the mean fluorescence intensity was performed by using the ZEN software (Zeiss, black edition). c Cell extracts were analyzed by RT-PCR for measuring the expression of N1 viral gene and the levels of the indicated cytokines. In parallel, the extract of uninfected cells was used as negative control.

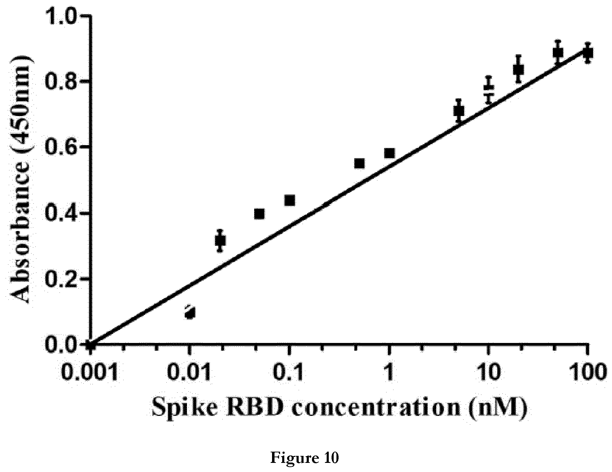

**Figure 10****: Detection of the levels of Spike protein in biological samples by using D3 mAb**. Immobilized ACE2 was used as capture molecule for Spike-RBD and the anti-Spike D3 mAb, was then added to detect the levels of the *spike antigen.* D3 mAb was used by testing known and increasing concentrations of RBD to obtain a calibration curve to be used for determining the levels of the antigen in the biological samples.

### Detailed description of the invention

### Selection of human scFvs specific for Spike of SARS-CoV-2

A novel selection strategy for the isolation of anti-Spike scFv-phages specific for RBD, in order to increase the probability to select mAbs capable of interfering in its interaction with ACE2, has been devised (Fig. 1a). To this aim, 4 panning rounds on human Spike RBD-Fc recombinant target protein were performed, followed by two parallel elution methods: classical acidic elution obtained by lowering the pH and a selective elution, by using the receptor ACE2-Fc chimeric protein which binds to Spike RBD with high affinity to elute those phages that specifically recognize the same epitope(see Fig. 1b). This approach was devised to guarantee an efficient selection of a large number of clones with a high specificity for RBD domain and mapping on the epitope recognized by ACE2, thus more likely endowed with the ability to interfere in the binding of Spike RBD to the ACE2 receptor, and capable of neutralizing the virus before its entry into the cells.

All the selection rounds consisted in two successive negative pannings on the Fc domain, followed by a positive selection on the human chimeric Spike RBD-Fc, to subtract from the collection the phages recognizing the Fc domain present in the chimeric protein. The eluted phages were used to infect *E*. *Coli* TG1 cells for amplification and further cycles of selection. The strategy of using different elution methods was aimed also at comparing the biological properties of the clones selected by the two strategies, in order to set up a procedure allowing for a novel epitope-specific selection strategy and for obtaining scFvs endowed with specific competitive functional properties.

The screening of positive clones was performed by ELISA and by Next Generation Sequencing (NGS) of the enriched pools of phages from each round of both the selections(see Fig. 1c-f).

### Screening by ELISA and expression of positive clones as soluble scFvs

The scFv phages from the 3^{rd} and 4^{th} rounds of both selections were tested by ELISA assays on immobilized Spike RBD protein for the screening of binders. In parallel, ELISA assays were performed also on immobilized human Fc domain to verify the specificity of the binders for RBD (see Fig. 2a).

As shown in Figure 2, four clones were found capable of specifically binding to RBD of Spike with no significant binding on the Fc, thus confirming the efficiency of the selection strategy. Sequence analyses of those clones revealed that at least two different scFvs sequences were selected, corresponding to the clones, named D3 and F12. To express the positive clones as soluble scFv proteins, the cDNA encoding each scFv was used to transform the bacterial strain SF110 and the expression was induced with isopropyl β-d-1-thiogalactopyranoside (IPTG). The periplasmic extracts of the two different selected clones were analyzed by Western Blotting²³ by using the anti-c-myc mAb for detection (Fig. 2b). As shown in Fig. 2b, the scFvs were expressed as soluble proteins of the expected molecular weight of 27 kDa.

### ELISA assays of scFvs on immobilized SARS-CoV-2 Spike RBD

To verify whether the soluble scFvs retained the binding properties of the scFvs displayed on phages, they were tested by ELISA assays, on Spike RBD-Fc chimeric protein, at increasing concentrations.

The selected anti-Spike soluble scFvs, named D3 and F12, were found able to selectively bind to RBD in a dose dependent manner (Fig. 3a).

To test whether the scFvs could also compete with the ACE2 receptor for the binding to S1 Spike protein, we performed an *in vitro* competitive ELISA assay²⁴ by measuring the binding of ACE2 to immobilized Spike RBD protein in the absence or in the presence of the selected soluble scFvs. As shown in Fig. 3b, the binding of ACE2 in the presence of D3 or F12 used as soluble scFvs was significantly reduced, compared to that observed in their absence, thus suggesting that the novel anti-Spike antibody fragments interfere in the interactions of Spike RBD with ACE2 receptor. To verify whether this ability could be confirmed also on ACE2-positive cells², *in vitro* competitive ELISA assays were performed on VERO E6 cells¹⁰, by measuring the binding of Spike RBD-Fc to the cells before or after a preincubation with a 5 fold molar excess of the selected soluble scFvs, for 2 hours at room temperature. As shown in Fig. 3c, the clones D3 and F12 interfere in the interaction of Spike RBD with ACE2 receptor also when it is expressed on the cell surface in its native conformation.

### Identification of enriched scFv sequences by Next-Generation Sequencing and conversion into antibodies

To quickly identify additional monoclonal antibodies targeting Spike protein from SARS-CoV-2, a high-throughput screening was implemented in parallel. The workflow was based on phage display coupled to next generation sequencing and to rapid conversion into fully human antibodies for binding and neutralization screening . Recombinant RBD-Fc of SARS-CoV-2 was used as bait for panning cycles and to widen the range of possible binders, two different elution methods were exploited in parallel selections. Thus, a non-specific pH-based elution was compared to a competitive one by using ACE2-Fc chimeric protein with the aim to reverse the interaction of those clones with a competitive binding for RBD. To identify potential binders, next generation sequencing was coupled to the screening to find out even those less represented clones within the enriched sub-libraries.

As the adopted phage library of scFvs consists of a few constant scaffold light chains, joined to highly diverse variable heavy chains, we extracted from all the selection cycles the VH region for further analysis of clonal diversity by NGS. Thus, plasmid dsDNA was extracted from phage-infected *E. Coli* cells from each round of selection for both elution methods. To discard empty scaffolds or fragmented clones (linker-VL, VH-linker), the 750 bp fragments corresponding to the full length inserts (VH-linker-VL) were isolated by digestion with suitable restriction enzymes. These purified fragments were further digested with *BamHI*, removing almost completely the VL region allowing to run samples in paired end 2×300 sequencing in MiSeq Illumina platform. VH fragments from different selection cycles were barcoded in pairs to pull in the same sequencing run cycle_1 & cycle_2 from competitive elution; cycle_1 & cycle_2 from pH elution; cycle_3 from acid & cycle_3 from competitive elution. The reads for each unique fragment were expressed as counts per million (cpm) to compare the trend of enrichment of a given sequence within and between the different selection cycles. Among the top 100 ranking clones, we discarded those that resulted out-of-frame as well as those bearing stop codons. In-frame sequences resulting from these filters, were translated into protein to merge cmp of clones with silent nucleotide changes. The heterogeneity and the overlapping between selection cycles were analysed. Eleven, 39, 7 and 2 unique in-frame sequences were identified respectively in the cycle 2, cycle 3 from competitive elution and cycle 2 and cycle 3 from acid elution. The cycle 3 from acid elution led to enrichment of non-informative clones (out-of-frame or stop codon-bearing) and dilution of only two potential binders among them. For this reason, cycle 2 from acid elution and cycle 3 from competitive elution resulted to be the best selection cycles in terms of diversity and numerosity. Interestingly, independently from elution method, 4 clones resulted commonly enriched into all the sub-libraries, including those corresponding to D3 and F12. Moreover, cycle 3 from competitive elution and cycle 2 from acid one, resulted highly enriched in two shared clones and in a plethora of sequences differing from the latter in only 1 amino acid position.

To improve the chance to identify clones targeting different epitopes of SARS-CoV-2 RBD, the only clones differing each other at least for two amino acids were considered for advanced analysis. Beyond the shared clones, we identified three clones specific for competitive elution, called S30, S33 and S96, and three as derived from acid elution, hereinafter referred to as AC1, AC2 and Ac3.

The trend of enrichment of these clones was analysed for both competitive and acid elution. Given the sharing of clones between competitive and acid elution, a total of 8 clones was identified as of interest (D3, F12, S30, S33, S96, AC1, AC2 and AC3) and were rescued by overlapping PCR with HCDR3-specific oligonucleotides from the enriched sub-libraries. The reconstituted scFvs were converted into fully human monoclonal antibodies by sub-cloning the variable domains in frame to human IgG4 Fc into mammalian expression vectors. Recombinant antibodies were produced and purified from HEK293EBNA_SINEUP_1 (abbreviated as HEK293ES_1).

### Characterization of the novel converted anti-Spike antibodies

The converted human IgG4 antibodies, generated from the scFvs screened by ELISA assays or identified by NGS as the best ranking Spike protein binders, were tested by ELISA assays on Spike RBD-Fc or Fc to confirm their binding specificity. As shown in Fig. 4, F12 and D3 mAbs retained the binding of the parental scFvs, already partially characterized. Among the 6 new identified enriched sequences by NGS, only two, named S96 and AC2, were found able to specifically bind to RBD, whereas the others did not significantly bind to RBD or recognized also the Fc.

We found that the best binder D3 specifically binds to the human RBD-Fc chimeric protein with high affinity (Kd 7.4 nM), while showing only a poor binding to the Fc domain used as a negative control (Fig. 5a).

To test its ability to interfere in the interaction between Spike RBD and ACE2 receptor, a competitive ELISA assay was also performed on immobilized Spike RBD by measuring increasing concentrations of ACE2-Fc in the absence or in the presence of 100nM of D3 mAb. The results, shown in Fig. 5b, indicate that the D3 mAb retains the ability of the parental scFv to interfere in the binding of ACE2 to Spike protein when used at a higher concentration than ACE2.

Similar results were obtained for the novel isolated F12 mAb, tested by ELISA assays on immobilized RBD to analyze its binding affinity and its ability to compete with ACE2 in the interaction with Spike. As reported in Fig. 5c, the antibody binds to the human RBD-Fc chimeric protein with a good affinity (Kd 15.5 nM) and significantly interferes in the binding of Spike to ACE2 receptor (see Fig. 5d).

Considering the competitive activity of both these two novel isolated anti-Spike mAbs, we combined them in the competitive ELISA assay, performed as described above. As reported in Fig. 5e, we confirmed that the combination of D3 and F12 antibodies show additive effects in the interference of Spike/ACE2 interaction.

The two additional antibodies, identified by NGS and named S96 and AC2, were found able to specifically bind to RBD with a lower affinity (Kd of about 26.5 nM and 45 nM for S96 and AC2, respectively) and to be less or not competitive with ACE2 for the binding to RBD with respect to F12 and D3 (see Fig. 6).

To test whether the different mechanisms of action of these two antibody groups is due to the different epitopes recognized, we performed competitive ELISA assays by measuring the binding to immobilized Spike RBD of biotinylated F12 in the absence or in the presence of saturating concentrations of unlabelled F12, D3, S96 or Ac2 mAbs. As shown in Fig. 8, the binding of biotinylated F12 in the presence of S96 or Ac2 mAb is not significantly reduced, whereas it is efficiently competed by unlabelled F12, used as a control, thus suggesting that the novel antibodies recognize different epitopes. The D3 mAb partially reduced (of about 25%) the binding of biotinylated F12 to RBD, thus suggesting that these two antibodies likely recognize distinct although overlapping epitopes.Cross-**reactivity of the novel anti-SARS-CoV-2 mAbs with SARS-CoV**

High sequence identity has been reported between the Spike proteins of SARS-CoV-2 and SARS-CoV, in particular in the RBDs where the amino-acid sequence identity reaches 73 %²⁵. Indeed, patients infected by SARS-CoV-2 were able to produce cross-reactive antibody responses to SARS-CoV RBD^{26, 13}.

To test whether the novel selected antibodies were cross-reactive for SARS-CoV RBD, we performed ELISA assays on SARS-CoV RBD by testing each mAb at increasing concentrations. As reported in Fig. 7, the novel anti-Spike F12 mAb does not recognize SARS-CoV RBD whereas the D3 and AC2 mAbs bind to SARS-CoV RBD, even if they show a lower binding ability with respect to that observed on SARS-CoV-2 RBD, tested in parallel assays as a positive control. On the contrary, the binding curve of S96 mAb on SARS-CoV RBD is superimposable to that obtained on SARS-CoV-2 RBD (Fig. 6a), thus suggesting that this antibody is highly cross-reactive for the other member of the Coronavirus family.

### SARS-CoV-2 neutralization by the novel human mAbs

The neutralizing activity of D3 was evaluated by preincubating increasing concentrations of the antibody (2-20 µg/ml concentration range) with the virus (0.005-0.5 MOI) for 1 hour at 37 °C. Then the mixtures were added to the VERO E6 cells to allow for the infection. After 60-70 hours of culture, the cells were harvested, washed and lysed for the extraction of RNA to be analyzed by RT-PCR for measuring the level of N1 viral gene into the cells.

As shown in Fig. 8a, the antibody D3 significantly inhibited the cell virus entry by totally blocking it at a concentration of 6 µg/mL when a low viral load was used and reducing it by 80% at a concentration of 20 µg/mL when a high viral load (0.5 MOI) was used. As shown in Fig. 8b, D3 efficiently inhibits the viral infectivity in a dose dependent manner, and to compare its effects with those of the other mAbs, we repeated the experiment by testing all of them at a concentration of 15 µg/mL (corresponding to the IC₅₀ of D3) with the infecting virus at the highest dose (0.5 MOI). As shown in Fig. 8c, S96 and F12 inhibited the viral infectivity by 30% and 40%, respectively, whereas AC2 did not show significant effects.

The lead neutralizing antibody D3 was further tested for its ability to inhibit the infectivity of cultures of human primary cells with the UK variant GISAID EPI_ISL_736997 and we found that the antibody retained its neutralizing ability (Fig. 9). In particular, D3 at a concentration of 50 nM significantly reduced the level of Spike in the infected cells, as shown by immunofluorescence analyses of treated cells. Since this UK variant contains the mutation D614G, implicated in increased transmissibility of up to 71% and multiple other mutations, including the substitution at position 501 in the RBD region (N501Y) and the following ones outside the RBD: H69del, V70del, Y144del, A570D, P681H, T716I, S982A, D1118H, the D3 antibody could be considered a potential therapeutic tool not only against the SARS-CoV2 virus but also against its variants.

### Lack of mAb toxicity in cell cultures

To assess potential toxic side effects of the novel anti-RBD mAbs in vitro, we performed cell viability and lysis analyses on VERO E6 cells treated with each neutralizing mAb for 72 h at 37°C. Cell viability tests were based either on counts by trypan blue or on 3-(4,5-dimethylthiazol-2-yl)-2,5- diphenyltetrazolium bromide test (MTT). Cell lysis was also checked by measuring lactate dehydrogenase (LDH) release in the culture medium. No significant toxic side effects were observed up to the highest concentration of 150 nM (about 25□g/mL). Furthermore, we quantified multiple cytokines in the extracts of D3-treated and infected human primary cells (Fig. 9) and we found that all the pro-inflammatory cytokines involved in tissue damages, such as IL-6 and TNFα, were significantly decreased by the treatment with D3, whereas the only cytokine observed at higher levels was IL-10 in line with its anti-inflammatory properties.

### D3 mAb can be used for the detection of Spike protein level by in biological samples

To test whether the novel mAbs could be used to develop a new rapid method to detect the level of Spike protein in the saliva biosamples to be used for diagnostic applications, we also set up an ELISA assay. Briefly, immobilized ACE2 was used as a capture molecule and the anti-Spike mAb, was then added to detect the levels of the *spike antigen. Thus*, the D3 mAb was used by testing known and increasing concentrations of RBD (see Fig. 10) to obtain a calibration curve to be used for determining the levels of the antigen in the saliva samples. Similar results were obtained also when the RBD or the whole S1 recombinant protein were heated at 65°C for 30 min to mimic the inactivation protocol of the virus in the saliva samples.

The novel scFvs obtained from both our selection strategies and devoid of the Fc, responsible for antibody-dependent enhancement (ADE) possible with human sera from convalescent patients, could be used in this format with no risks of inducing effector functions. However, to increase their stability and avidity we converted them also into full size IgG4, differently from the previously reported IgG1 mAbs, to avoid unwanted potential harmful side effects of IgG1 endowed with potent effector functions on immune system.

We found that the novel isolated antibodies were able to specifically bind to RBD domain in a nanomolar range. The isolates D3 and F12 can interfere in the interaction of Spike protein with ACE2 receptor, either used as purified protein or when expressed on cells in its native conformation. Moreover, by competitive ELISA assays we demonstrated that some of them recognize different epitopes.

When tested for their inhibitory effects on cultures of VERO E6 cells infected with the virus, the antibody D3 displayed the highest neutralization potency, by totally blocking cell virus entry at a concentration of 6 µg/mL when a low viral load was used and by reducing it by 80% at a concentration of 20µg/mL when a high viral load (0.5 MOI) was used. Interestingly, the only antibody devoid of biological activity against SarsCoV2 was found to be AC2, the antibody obtained by the acidic elution of phages, thus suggesting that the novel strategy of competitive elution with ACE2 allowed us to identify not only a larger number of binders but also those endowed with biological effects against the virus, thus confirming the efficiency of our strategy.

Recently many reports in literature have described viral mutations, selected during treatment of infected patients or spreading globally to confer resistance, that could render ineffective the treatment with antibodies³¹⁻³³. In particular, the UK variant with the mutation D614G, implicated in increased transmissibility of up to 71% over and above the previous circulating strains of SARS-CoV-2, was derived from the SARS-CoV-2 20B/GR clade (lineage B.1.1.7) and contains multiple mutations, including the substitution at position 501 in the RBD of viral S gene (N501Y) which is the target of the novel proteins of the invention ^{8, 34-36}.

Thus, we further tested D3 for its ability to inhibit the infectivity of cultures of human primary cells with the UK variant and we found that the antibody retained its neutralizing ability with no side toxic or pro-inflammatory effects, thus confirming its potential as a safe therapeutic tool.

In order to test whether the novel antibodies could be used also for inhibiting other members of Coronavirus family, we tested them for their binding to Spike-RBD of SARS-CoV virus and we found that one of them (S96) showed significant cross-reactivity.

### Materials and Methods

### Bacterial strains, Culture Media and Antibiotics

*E.Coli TG1* bacterial strain was used for the production of phages; *E.Coli SF110* bacterial strain was used for the expression of the scFvs as soluble proteins. Bacterial growth was carried out in the culture media YT-Medium (A0981) and LB (A0954) from AppliChen Darmstadt, Germany. The Antibiotics used were ampicillin (A7492) at the concentration of 100 µg/ml and kanamycin (A1493) at the concentration of 25 µg/ml (from AppliChen Darmstadt Germany).

### Eukaryotic cell cultures

VERO E6 cells were cultured in Dulbecco's Modified Eagle's Medium (GibcoTM DMEM, Thermo Fisher Scientific 11965 Ferentino Italy), supplemented with 10% (vol/vol) heat-inactivated fetal bovine serum (FBS, Sigma-Aldrich F2442, USA), 50 UI mL⁻¹ Penicillin Streptomycin (Gibco Life Technologies 15140-122, Grand Island USA) and 2 nM L-glutamine (Gibco Life Technologies 25030-024, Paisley UK). Freshly isolated human nasal epithelial cells were collected by nasal brushing of healthy donors. The ACE2 gene locus of the nasal epithelial cells underwent next-generation sequencing and was shown not to have any modifier variants (synonymous_variant:c.2247G>A,p.Val749Val;intron_variant:c.1297+68_1297+69insCT T;splice_region_variant&intron_variant:c.439+4G>A; whole exome sequencing data deposited with the European Variation Archive (EVA) - EMBL-EBI; Project ID: PRJEB42411; Analyses: ERZ1700617). These cells were cultured in PneumaCult (#05009; StemCell Technologies USA) with 2 mM L-glutamine (Gibco Life Technologies 25030-024, Paisley UK) and 1% penicillin/streptomycin (P0781; Sigma USA). Cell cultures were grown at 37°C as previously described³⁷.

### Antibodies and human recombinant proteins

The following human recombinant proteins were used: SARS-CoV-2 (2019-nCoV) chimeric Spike RBD-Fc protein, SARS-CoV-2 (2019-nCoV) Spike RBD-His protein and ACE2 His Protein (all from Sino Biological, 10108-H08H Düsseldorfer Eschborn, Germany). Recombinant ACE-2 Fc chimeric protein (Z03484 GenScript, Piscataway NJ); recombinant IgG1 Fc protein (R & D Systems, 110-HG Minneapolis MN).

The following antibodies were used: HRP conjugated anti-His antibody (Qiagen, 1014992 Hilden, Germany); HRP conjugated anti-cmyc antibody (Milteny Biotec, 130-092-113 GmbH Friedrich-Ebert-Straβe Germany); HRP conjugated anti-human Fc antibody (Sigma, AP113P USA); anti-human IgG (Fab')2 goat monoclonal antibody (Abcam, Cambridge UK ab98535).

### Selection of scFv-phage clones

Phagemid particles were isolated from the library by using the M13-K07 helper phage (Invitrogen, Thermo Fisher Scientific Carlsbad, CA 92008, USA), as previously reported³⁸. To isolate anti-Spike scFv-phages, a phagemid library of up 10¹² different clones was used for selection on human chimeric Spike RBD-Fc protein³⁹. For each round of selection, phages were firstly blocked with 5% (wt/vol) Skim Milk Powder in PBS (Fluka Analytical, Sigma-Aldrich, A08300500 USA) and then two negative pannings were performed by incubating them on immobilized rhIgG1-Fc protein domain in 2.5% (wt/vol) Skim Milk Powder for 2 hours at 4°C by gently rotation. The unbound phages were collected in the supernatant by centrifugation at 1200 rpm for 10 minutes and then incubated with immobilized Spike RBD-Fc protein (20 µg/ml) in 2.5% (wt/vol) Skim Milk Powder over night at 4°C by gently rotation for the positive selection. After extensive washes with PBS, the bound phages were eluted from the Spike RBD-Fc protein by using two parallel elution methods: the classical elution with 76 mM citric acid (pH 2.5) in PBS for 5 minutes followed by the addition of 1M Tris-HCl (pH 8.0) neutralization buffer^{21, 39}, or selective competitive elution, by using the chimeric receptor ACE2-Fc (100µg/ml in PBS) which binds to Spike-RBD with high affinity to obtain those phages that specifically recognize the same epitope. The eluted phages were used to infect *E. coli* TG1 cells for amplification and further rounds of selection on the purified chimeric protein. Finally, the collected phages were stored at 4°C until use.

### Screening of positive clones by ELISA assays

To prepare phages for screening of positive clones by ELISA assay, TG1 cells were infected with the phages eluted from the 3^{rd} and 4^{th} rounds of selection and plated on 2xTY/agar containing glucose (1%) and ampicillin (100 µg/ml) to obtain single separated bacterial clones. The clones were picked and grown into 96-well plates in 2xTY medium containing 1% glucose and ampicillin (100 µg/ml) for 18 hours in agitation at 37°C. A superinfection with M13-K07 helper phage was performed to produce the scFv-phages, that were used for ELISA assays on immobilized Spike RBD-Fc chimeric protein, as previously reported^{23, 38,40}.

### Analysis of positive clones and expression of soluble scFvs

The positive clones selected by ELISA assays were grown in 2xTY medium containing glucose (1%) and ampicillin (100 µg/ml) to extract the plasmidic DNA by using QIAprep Spin Miniprep Kit (QIAGEN, 27106 Venlo Netherlands). To determine the number of positive clones containing the cDNA encoding the full length scFv, the selected plasmidic DNA was digested from pHEN2 vector with *NcoI* and *NotI* restriction enzymes (R3193L and R3189L, respectively from New England Biolabs, Massachusetts USA) and analyzed by electrophoresis on 1% agarose gel. The positive clones showing the full length insert were prepared for sequencing by using the LMB3 forward and the fdSeq1 reverse primers, respectively complementary to the sequence located upstream the heavy variable (VH) region sequence and downstream to the c-myc-tag sequence.

To express the selected positive clones as soluble scFv molecules, *E. Coli* SF110 cells were infected with each of the anti-Spike scFv-phage preparations and grown in 2xTY medium containing ampicillin (100 µg/ml). To express soluble scFv, the Isopropyl β-D-1-thiogalactopyranoside (IPTG AppliChen A4773, Darmstadt Germany) was added at the concentration of 1 mM (16 hours at R T) when the optical density of the cell culture reached the absorbency of 0.8. Cells were harvested by centrifugation at 3000 rpm for 20 minutes and resuspended in *B-PER* (ThermoFisher 78248 Meridian Rockford USA) for 20 minutes by gently rotation at room temperature (RT). Cell suspensions were then centrifuged at 12000 rpm for 30 minutes at 4°C to obtain the periplasmic extract. Soluble scFvs were analyzed by Western Blotting detected by using a mouse HRP-conjugated anti-c-myc-tag monoclonal antibody.

### DNA fragment preparation and libraries generation for high-throughput sequencing

For each cycle, the scFv-containing double strand DNAs were purified from superinfected *E. coli* TG1 cell cultures using Endo free Plasmid Maxi Kit (Qiagen, 12362, Hilden Germany). Heavy chain fragments were isolated by two-step restriction process, as previously described²⁴. Briefly, full-length scFvₛ (VH-Linker-VL) were extracted by using *Nco*I (R3193L New England Biolabs, Ipswich, Massachusetts, USA) and *Not*I (R3189L New England Biolabs, Massachusetts USA) and purified from 1.5% agarose gel using Wizard SV Gel and PCR Clean-Up System (Promega, A9282, Wisconsin USA). To generate fragments suitable for Next Generation Sequencing (NGS), a portion of variable light (VL) chain was removed from full-length scFvₛ by digestion with *Bam*HI (R3136S New England Biolabs, Massachusetts USA) and the obtained fragment was purified from 1.5% agarose gel using Wizard SV Gel and PCR Clean-Up System (Promega, REF A9282, Wisconsin USA).

Library preparation for sequencing and preliminary bioinformatic analysis of the data were performed at the Center for Translational Genomics and Bioinformatics, Hospital San Raffaele, Milano, Italy, as previously reported^{41, 42}.

Briefly, isolated VH fragments from each cycle were bar-coded by TruSeq ChIP sample prep kit (Illumina, 15023092) and sequenced to a final concentration of 10 pM with 2 × 300 nt SBS kit v3 on an Illumina MiSeq apparatus. For each sub-library, raw counts were normalized to the total number of counts, obtaining count per million (cpm) values. Obtained nucleotide sequences were translated, taking into account the correct open reading frame.

### scFv reconstitution, antibodies production and purification

The clones of interest were isolated from competitive elution cycle_3 and acidic elution cycle_2 by overlapping PCR, as previously described⁴¹. Briefly, to obtain separate VH and VL fragments, two different PCR reactions were performed by using Phusion High-Fidelity DNA Polymerase (Thermo Fisher Scientific, F530S, Massachusetts USA) and clone-specific primers designed within each HCDR3 and in the constant region of plasmid upstream and downstream of VH and VL. To obtain full-length scFvₛ, VH and VL fragments were mixed and extended by HCDR3 overlapping PCR. Recovered scFvₛ were converted into whole human IgG4 antibodies by cloning the corresponding VH and VL cDNAs in the SINEUP-competent 8.2VH and 4.2VL pEU vectors, expressing the constant heavy and light chains respectively⁴³. Briefly, VHs and VLs were amplified by CloneAmp HiFi PCR Premix using specific oligos. In-Fusion HD cloning kit (Clontech Laboratories, 639692, California USA) was used to clone VH in *Bam*HI (R3136S) and *Bss*HII (R0119L) linearized pEU8.2VH vector, and VL in *Apa*LI (R0507L) and *Avr*II (R0174) linearized pEU4.2VL vector. All restriction enzymes were from New England Biolabs, Massachusetts USA.

The obtained vectors were transformed into Stellar Competent Cells (Clontech Laboratories, 636763, California USA) and the resulting colonies were screened by sequence analysis. The vectors containing the correct DNA sequences were prepared with an endotoxin-free system (EndoFree Plasmid Maxi Kit, Qiagen, 12362).

Antibodies were produced co-transfecting VH and VL expressing vectors by using Lipofectamine Transfection Reagent (LifeTechnologies, Inc. 11668019, California USA) into the production enhanced cell line HEK293EBNA SINEUP (HEK293_ES1), expressing the long non-coding SINEUP targeting heavy and light chain signal peptide on mRNAs⁴³. Transfected cells were grown for 10 days at 37 °C in CHO medium (Gibco, LifeTechnologes, Inc. 10743029, Waltham, Massachusetts, USA) supplemented with 1% L-glutamine 200 mM (Gibco, LifeTechnologies, A2916801, Massachusetts USA) and 1% Penicillin-Streptomycin 10,000 U/mL (Sigma-Aldrich, P0781, Missouri USA). The antibodies were purified from the conditioned media by using Protein A HP SpinTrap30 (GE Healthcare Life Science, 28-9031-32, Chicago USA), following manufacturer's instruction. Desalting and buffer-exchange were performed by using PD-10 Column (GE Healthcare Life Sciences, 17085101, Chicago, Illinois, USA). Antibodies were sterilized by filtration with 0.22 µm durapore hydrophilic filters (Millipore, SLGS033SS, Massachusetts USA) and stored in aliquots at -80 °C.

### ELISA assays

To confirm the binding specificity of the novel anti-Spike soluble scFvs or purified antibodies, ELISA assays were performed on the chimeric Spike RBD-Fc protein. The NuncTM flat bottom 96-well plates (Thermo Fisher Scientific 439454 Ferentino Italy) were coated with 5 µg/mL Spike RBD-Fc recombinant protein in a solution of 0.05 M NaHCO3 for 72 hours at 4°C. After blocking of the coated 96-well plates with 5% nonfat dry milk in PBS for 1 hour at 37°C, the D3 and F12 soluble scFvs or purified mAbs were added at increasing concentrations to the plates in 3% Bovine Serum Albumin (BSA Sigma A7030, St Louise MO) in PBS and incubated for 2 hours at RT by gently shaking. After the first incubation, extensive washes were carried out with PBS, and the plates were incubated for 1 hour at RT with anti-cmyc or anti-Fab HRP-conjugated antibody, for the detection of soluble scFvs or antibodies, respectively. After washes the plates were incubated with 3,3',5,5'-tetramethylbenzidine (TMB Sigma-Aldrich T0440, St Louise MO) reagent, as previously reported^{24,44,45.}. Absorbance at 450 nm was measured by the Envision plate reader (Perkin Elmer, 2102). Binding values were reported as the mean of at least three determinations obtained in three independent experiments^{44, 45}.

The Kd values were calculated by the analysis of binding curves of the selected antibodies with the Graphpad Prism software, as previously reported²⁴. The equation model used was: Y=Bmax^{∗}X/(Kd+X) + NS^{∗}X + Background. Bmax is the maximum specific binding in the same units as Y; Kd is the equilibrium binding constant, in the same units as X, and it is the ligand concentration needed to achieve a half-maximum binding at equilibrium; NS is the slope of non-specific binding in Y units divided by X units; background is the amount of nonspecific binding with no added ligand²⁴.

### Competitive ELISA assays

In order to investigate the ability of the novel isolated D3 and F12 scFv clones to interfere in the binding of Spike protein to ACE2 receptor, competitive ELISA assays were performed on immobilized Spike RBD-Fc protein or on ACE2-positive VERO E6 cells. To this aim, the ELISA assays on chimeric protein were performed by coating 5ug/ml of Spike RBD-Fc recombinant protein as described above in 0.005M NaHCO₃ solution in a NuncTM flat-bottom 96-well plate for 72 hours at 4°C.

D3 or F12 soluble scFvs, preincubated (5 fold molar excess) with ACE2-His recombinant protein for 2 hours at RT, were added to the coated plate for 2 hours in agitation at RT. The plates were washed with PBS and the binding of ACE2 to immobilized Spike protein, in the absence or in the presence of the selected soluble scFvs, was measured by incubating the plate with the HRP conjugated anti-His antibody for 1 hour in agitation at RT. After washes TMB reagent was added and the signals analyzed as described above. To test the ability of the selected clones to interfere in the binding of Spike protein to ACE2 receptor expressed on VERO E6 cells, cell ELISA assays were performed by plating the cells in round-bottom 96-well plates (2 x 10⁵ cells or for each well). The D3 or F12 soluble scFvs were preincubated with Spike-Fc protein (at 5 fold molar excess) for 2 hours at RT and then added to the cells for 2 hours in agitation at RT. After washes the binding of Spike to the cells, in the absence or in the presence of the selected soluble scFvs, was measured by incubating the plate with the HRP conjugated anti-IgG Fc antibody, and analyzed as described above.

To confirm the ability of the converted IgG4 antibodies to interfere in the interaction between Spike RBD and ACE2 receptor, the competitive ELISA assays were also performed on immobilized Spike RBD-Fc recombinant protein by measuring increasing concentrations of ACE2-His, in the absence or in the presence of each mAb. After blocking, the coated plates were preincubated with the mAbs used at the concentration of 100 nM for 2 hours in agitation at RT. Then, increasing concentrations of ACE2-His recombinant protein were incubated for 2 hours in agitation. The plates were washed and the binding of ACE2-His was measured by incubating the plate with the HRP-conjugated anti-His antibody for 1 hour in agitation at RT, and analyzed as described above.

To evaluate whether the epitopes recognized by the novel isolated anti-Spike mAbs are different, competitive ELISA assays were performed on immobilized Spike RBD-Fc protein (5 µg/mL) by measuring the binding of the biotinylated F12 mAb (Linghtning-Link Biotin conjugation Kit Type B, Innova Biosciences 715-0010, Cambridge UK) to the protein. The biotinylated F12 mAb was tested at increasing concentrations, in the absence or in the presence of saturating concentrations of unlabelled D3, S96 or AC2 mAbs (300 nM) preincubated over night at 4°C. After washes, the binding of the biotinylated F12 mAb was detected with HRP-conjugated Streptavidin (Biorad 710005, Kidlington UK) and the absorbance measured as reported above.

### In-vitro infection

SARS-CoV-2 viral particles were obtained from a frozen swab from two patients positive for COVID-19 (**Italian Variant**, Genbank MT682732.1, GISAID EPI_ISL_477204; **UK variant,** GISAID EPI_ISL_736997). SARS-CoV-2 particles at different multiplicity of infection (i.e. 0.05 - 0.5 MOI) were preincubated with the antibodies (D3, F12, S96, AC2) at 37°C for 1 hour and then added to Vero E6 (8 x 10⁵/flask) or human primary nasal epithelial cells (3 x 10⁵/flask). Non-infected cells were used as the negative infection control. After 72 h of infection, the cells were lysed and their RNA was extracted. These experiments were performed in a BLS3 authorised laboratory.

***The Spike in UK variant contains the following mutations:*** H69del, V70del, Y144del, N501Y, A570D, D614G, P681H, T716I, S982A, D1118H.

### Real-time RT-PCR assays

### N1-3 detection

RNA samples were extracted with TRIzol RNA Isolation Reagent (#15596018; Ambion, Thermo Fisher Scientific), according to the manufacturer instructions. Real-time RT-PCR was performed using 'quanty COVID-19' kits (Ref. RT-25; Clonit; US Food and Drug Administration *'in-vitro* diagnostic' (IVD) approved). These kits allow specific quantitative detection of the N1, N2 and N3 fragments (from the SARS-CoV-2 N gene), using differentially labelled target probes. These runs were performed on a PCR machine (CFX96; BioRad) under the following conditions: 25 °C for 2 min; 50 °C for 15 min; 95 °C for 2 min; 95 °C for 3 s; 55 °C for 30 s (×45 cycles). Cq values of N1, N2 and N3 were calculated as means ±standard deviation, as the ratios to the internal control detected with the CLONIT quanty COVID-19 kits.

### N1 and cytokines detection

RNA samples were extracted with TRIzol RNA Isolation Reagent (#15596018; Ambion, Thermo Fisher Scientific), according to the manufacturer instructions. Reverse transcription was carried out by using '5 × All-in-one RT Mastermix' (#g486; ABM), following the manufacturer instructions. The cDNA preparation was analyzed through the cycling method, as follows: incubation of the complete reaction mix at 25 °C for 5 min; at 42 °C for 30 min; at 85 °C for 5 min; and hold at 4 °C. The reverse transcription products (cDNA) were amplified by quantitative real-time PCR using a real-time PCR system (7900; Applied Biosystems, Foster City, CA, USA). The relative expression of the target genes was determined using the 2-ΔΔCt method as fold over vehicle-SARS-CoV-2 infected control. The data are presented as 2-ΔΔCt means ±standard deviations of two to three replicates. The target genes were detected using a Brightgreen 2× qPCR Mastermix low-rox (#Mastermix-lr; ABM.). The details of the primers used in these assays are listed:

| **Gene** | **Primer** | **Primer sequence** |
|---|---|---|
| N1* | Forward | GACCCCAAAATCAGCGAAAT SEQ ID 9 |
| | Reverse | TCTGGTTACTGCCAGTTGAATCTG SEQ ID 10 |
| IL-6 | Forward | GCCACTCACCTCTTCAGAAC SEQ ID 11 |
| | Reverse | AGCATCCATCTTTTTCAGCC SEQ ID 12 |
| IL-10 | Forward | CCTGCCTAACATGCTTCGAGA SEQ ID 13 |
| | Reverse | TGTCCAGCTGATCCTTCATTTG SEQ ID 14 |
| IL-12 | Forward | TGATGGCCCTGTGCCTTAGT SEQ ID 15 |
| | Reverse | GGATCCATCAGAAGCTTTGCA SEQ ID 16 |
| IFN-γ | Forward | AGGCATTTTGAAGAATTGGAAAGA SEQ ID 17 |
| | Reverse | AGTAAAAGGAGACAATTTGGCTCT SEQ ID 18 |
| TNF-α | Forward | TCTCTCTAATCAGCCCTCTGG SEQ ID 19 |
| | Reverse | GCTACATGGGCTACAGGC SEQ ID 20 |

| | | |
|---|---|---|
| * approved by CDC | | |

### Immunofluorescence

Cells (1 × 10⁴) were fixed in 4% paraformaldehyde in PBS for 30 min. washed three times with PBS, and permeabilised with 0.1% Triton X-100 (215680010; Acros Organics Rodano Italy) diluted in PBS, for 15 min. The cells were then washed with PBS and blocked with 3% bovine serum albumin (A9418; Sigma) in PBS for 1 h at room temperature. The slides were incubated with the relevant primary antibodies overnight at 4 °C: anti-ACE2 (1:50; ab15348; Abcam Cambridge UK) and anti-SARS Spike (1:50; ab272420; Abcam). After washing twice with PBS, the samples were incubated with the relevant secondary antibody at room temperature for 1 h: anti-mouse Alexa Fluor 488 (1:100; ab150113; Abcam) and anti-Rabbit Alexa Fluor 647 (1:100; ab150075; Abcam). DNA was stained with DAPI (1:5000; #62254; Thermo Fisher). The slides were washed and mounted with cover slips using 50% glycerol (G5150; Sigma-Aldrich USA). Microscopy images were obtained with the Elyra 7 platform (Zeiss) with the optical Lattice SIM technology, using the 63× oil immersion objective. The quantification of the fluorescence intensity was performed using the ZEN software (Zeiss, black edition).

### In vitro cytotoxicity assays

To test the cytotoxic effects of the novel generated IgG4 anti-Spike mAbs, VERO E6 cells were plated in 96-well flat bottom plates at the density of 2 × 10⁵ cells/well and incubated for 16 h at 37 °C. The mAbs, used at a high concentration (150 nM), were added in the complete culture medium and incubated for 72 h. After treatment, the supernatants were analyzed for measuring LDH release and cell viability was measured by the trypan blue exclusion test³⁷. Cell survival was expressed as percent of viable cells in the presence of the mAbs with respect to the control cells untreated or treated with an unrelated IgG4, used as a negative control.

### Statistical Analyses

Error bars were calculated on the basis of the results obtained by at least three independent experiments and represent means ± SD. For these studies, differences between the experiments were assessed by two-tailed Student's t-test and the statistical significance was defined as *** p ≤0.001; ** p <0.01; * p <0.05. A probability (P) <0.05 was considered statistically significant.

### References

1. Bourgonje, A.R. et al. Angiotensin-converting enzyme 2 (ACE2), SARS-CoV-2 and the pathophysiology of coronavirus disease 2019 (COVID-19). J Pathol. 251, 228-248 (2020).
2. Lu, C.Y. et al. siRNA silencing of angiotensin-converting enzyme 2 reduced severe acute respiratory syndrome-associated coronavirus replications in Vero E6 cells. Eur J Clin Microbiol Infect Dis. 27, 709-15 (2008).
3. Phua, J. et al. Asian Critical Care Clinical Trials Group. Intensive care management of coronavirus disease 2019 (COVID-19): challenges and recommendations. Lancet Respir Med. 8, 506-517 (2020).
4. Uhal, B.D. et al. Cell cycle dependence of ACE-2 explains downregulation in idiopathic pulmonary fibrosis. Eur Respir J. 42, 198-210 (2013).
5. Isaacs, D. et al., M.R. Epidemiology of coronavirus respiratory infections. Arch Dis Child. 58, 500-3 (1983).
6. Su, S. et al. Epidemiology, Genetic Recombination, and Pathogenesis of Coronaviruses. Trends Microbiol. 24, 490-502 (2016).
7. Du, L. et al. The spike protein of SARS-CoV--a target for vaccine and therapeutic development. Nat Rev Microbiol. 7, 226-36 (2009).
8. Tai, W. et al. Characterization of the receptor-binding domain (RBD) of 2019 novel coronavirus: implication for development of RBD protein as a viral attachment inhibitor and vaccine. Cell Mol Immunol. 17, 613-620 (2020).
9. Gui, M. et al. Cryo-electron microscopy structures of the SARS-CoV spike glycoprotein reveal a prerequisite conformational state for receptor binding. Cell Res. 27, 119-129 (2017).
10. Walls, A.C. et al. Tectonic conformational changes of a coronavirus spike glycoprotein promote membrane fusion. Proc Natl Acad Sci USA. 114, 11157-11162 (2017).
11. Wong, S.K. et al., A 193-amino acid fragment of the SARS coronavirus S protein efficiently binds angiotensin-converting enzyme 2. J Biol Chem. 279, 3197-201 (2004).
12. Tortorici, M.A., Veesler, D. Structural insights into coronavirus entry. Adv Virus Res. 105, 93-116 (2019).
13. Bosch, B.J. et al., The coronavirus spike protein is a class I virus fusion protein: structural and functional characterization of the fusion core complex. J Virol. 77, 8801-11 (2003).
14. Wang, M. et al. Remdesivir and chloroquine effectively inhibit the recently emerged novel coronavirus (2019-nCoV) in vitro. Cell Res. 30, 269-271 (2020).
15. Magro, G. COVID-19: Review on latest available drugs and therapies against SARS-CoV-2. Coagulation and inflammation cross-talking. Virus Res. 286, 198070 (2020).
16. Li, H. et al. Updated Approaches against SARS-CoV-2. Antimicrob Agents Chemother. 64, e00483-20 (2020).
17. CEPI. (2020).
18. Oliver, S.E. et al. The Advisory Committee on Immunization Practices' Interim Recommendation for Use of Pfizer-BioNTech COVID-19 Vaccine - United States, December 2020. MMWRMorb Mortal Wkly Rep. 69, 1922-1924 (2020).
19. Watanabe, Y. et al. Native-like SARS-CoV-2 spike glycoprotein expressed by ChAdOx1 nCoV-19/AZD1222 vaccine. bioRxiv [Preprint]. 2021.01.15.426463 (2021).
20. Dyer, O. Food and Drug Administration. Letter to Eli Lilly and Company (2020).
21. Noy-Porat, T. et al. A panel of human neutralizing mAbs targeting SARS-CoV-2 spike at multiple epitopes. Nat Commun. 11, 4303 (2020).
22. Lan, J. et al. Structure of the SARS-CoV-2 spike receptor-binding domain bound to the ACE2 receptor. Nature. 581, 215-220 (2020).
23. De Lorenzo, C. et al., G. A new human antitumor immunoreagent specific for ErbB2. Clin Cancer Res. 8, 1710-9 (2002).
24. Sasso, E. et al..,Massive parallel screening of phage libraries for the generation of repertoires of human immunomodulatory monoclonal antibodies. MAbs. 10, 1060-1072 (2018).
25. Tian, X. et al. Potent binding of 2019 novel coronavirus spike protein by a SARS coronavirus-specific human monoclonal antibody. Emerg Microbes Infect. 9, 382-385 (2020).
26. Lv, H. et al. Cross-reactive Antibody Response between SARS-CoV-2 and SARS-CoV Infections. Cell Rep. 31, 107725 (2020).
27. Chung, J.Y., Thone, M.N.; Kwon, Y.J. COVID-19 vaccines: The status and perspectives in delivery points of view. Adv Drug Deliv Rev. 170, 1-25 (2020).
28. Chi, X. et al. A neutralizing human antibody binds to the N-terminal domain of the Spike protein of SARS-CoV-2. Science. 369, 650-655 (2020).
29. Jahanshahlu, L., Rezaei, N. Monoclonal antibody as a potential anti-COVID-19. Biomed Pharmacother. 129, 110337 (2020).
30. Diseasesm A. V. et al., Reaction of Human Monoclonal Antibodies to SARS-CoV-2 Proteins With Tissue Antigens: Implications for Autoimmune. Frontiers in Immunology. 11, 617089 (2021).
31. Forster, P. et al., Phylogenetic network analysis of SARS-CoV-2 genomes. Proc Natl Acad Sci USA. 117, 9241-9243 (2020).
32. Weisblum, Y. et al. Escape from neutralizing antibodies by SARS-CoV-2 spike protein variants. Elife. 9, e61312 (2020).
33. Hu, J. et al., Genetic variants are identified to increase risk of COVID-19 related mortality from UK Biobank data. medRxiv [Preprint]. Nov 9, 2020.11.05.20226761 (2020).
34. Tang, J.W., Tambyah, PA., Hui, D.S. Emergence of a new SARS-CoV-2 variant in the UK. J Infect. S0163-4453(20)30786-6 (2020).
35. Volz, E. et al. Evaluating the Effects of SARS-CoV-2 Spike Mutation D614G on Transmissibility and Pathogenicity. Cell. 184, 64-75.e11 (2021).
36. Nicholas, G. et al. Estimated transmissibility and severity of novel SARS-CoV-2 Variant of Concern 202012/01 in England. medRxiv 12.24.20248822 (2020).
37. Gelardi, T. et al. Two novel human anti-ErbB2 immunoagents are active on trastuzumab-resistant tumours. Br J Cancer. 102, 513-9 (2010).
38. Palmieri, D. et al. Human anti-nucleolin recombinant immunoagent for cancer therapy. Proc Natl Acad Sci USA. 112, 9418-23 (2015).
39. Passariello, M. et al. Isolation of Two Novel Human Anti-CTLA-4 mAbs with Intriguing Biological Properties on Tumor and NK Cells. Cancers (Basel). 12, 2204 (2020).
40. Paciello, R. et al. Novel human anti-claudin 1 mAbs inhibit hepatitis C virus infection and may synergize with anti-SRB1 mAb. J Gen Virol. 97, 82-94 (2016).
41. Sasso, E. et al. One-step recovery of scFv clones from high throughput sequencing-based screening of phage display libraries challenged to cells expressing native Claudin-1. BioMed Res Int. 2015, 703213 (2015).
42. Cembrola, B. et al. Rapid Affinity Maturation of Novel Anti-PD-Ll Antibodies by a Fast Drop of the Antigen Concentration and FACS Selection of Yeast Libraries. Biomed Res Int, 2019, 6051870 (2019).
43. Sasso, E. et al. A long non-coding SINEUP RNA boosts semi-stable production of fully human monoclonal antibodies in HEK293E cells. MAbs. 10, 730-737 (2018).
44. Passariello, M. et al. Novel Human Anti-PD-Ll mAbs Inhibit Immune-Independent Tumor Cell Growth and PD-L1 Associated Intracellular Signalling. Sci Rep. 9, 13125 (2019).
45. Passariello, M., et al., Novel Human Bispecific Aptamer-Antibody Conjugates for Efficient Cancer Cell Killing. Cancers (Basel). 11, 1268 (2019).

## Claims

1. An antigen specific protein binding to SARS-CoV2 Spike RBD comprising:
i. a heavy chain variable region comprising an amino acid sequence selected from SEQ ID NO: 1, SEQ ID NO 3 and SEQ ID NO 4 and
ii. a light chain variable region comprising the amino acid sequence SEQ ID NO 2.

2. An antigen specific protein according to claim 1, which is a human antibody, a humanized antibody, a chimeric antibody, a monoclonal antibody, a multispecific antibody, a recombinant antibody, an antigen-binding antibody fragment, a single chain antibody, a single chain variable fragment antibody, a diabody, an antibody-like protein or a fragment thereof, a Fab fragment, an F(ab)2 fragment, an antibody mimetic, an IgG1 antibody, an IgG2 antibody, an IgG3 antibody or an IgG4 antibody.

3. An antigen specific protein according to claim 1 which is an IgG4 antibody.

4. An antigen specific protein according to any one of claims 1 to 3 specifically binding to Spike RBD and neutralizing the SARS-CoV2 cell entry and infection.

5. A method for the preparation of the antigen specific proteins of claims 1-3, comprising the step of isolating anti-Spike scFv-phages specific for RBD by panning phages on human Spike RBD-Fc recombinant target protein followed by a selective elution on an excess of receptor ACE2-Fc chimeric protein which binds to Spike RBD.

6. A nucleotide sequence coding for the antigen specific proteins of claims 1-4.

7. A pharmaceutical composition comprising an antigen specific protein of claims 1-4 or the nucleotide sequence of claim 6.

8. A method for the detection of Sars-Cov 2 spike antigen in a biological sample comprising incubating said sample with an antigen specific protein of claims 1-4 and measuring the binding of said antigen to said antigen specific protein.

9. A method according to claim 8 wherein the antigen specific protein is measured is labelled by an enzyme, a fluorophore or chromophore.


SEQUENCE LISTING

<110>  CEINGE BIOTECNOLOGIE AVANZATE SCARL

<120>

<130>  3603eur

<160>  20

<170>  PatentIn version 3.5

<210>  1
<211>  117
<212>  PRT
<213>  Homo sapiens

<400>  1

Gln Val Gln Leu Val Gln Ser Gly Ala Glu Val Lys Lys Pro Gly Ala
1               5                   10                  15

Thr Val Lys Ile Ser Cys Lys Val Ser Gly Tyr Thr Phe Thr Asp Tyr
            20                  25                  30

Tyr Met His Trp Val Gln Gln Ala Pro Gly Lys Gly Leu Glu Trp Met
        35                  40                  45

Gly Leu Val Asp Pro Glu Asp Gly Glu Thr Ile Tyr Ala Glu Lys Phe
    50                  55                  60

Gln Gly Arg Val Thr Ile Thr Ala Asp Thr Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Ala Lys Ala Thr Leu Gly Leu Trp Gly Gln Gly Thr Leu
            100                 105                 110

Val Thr Val Ser Arg
        115

<210>  2
<211>  106
<212>  PRT
<213>  Homo sapiens

<400>  2

Glu Leu Thr Gln Asp Pro Ala Val Ser Val Ala Leu Gly Gln Thr Val
1               5                   10                  15

Arg Ile Thr Cys Gln Gly Asp Ser Leu Arg Ser Tyr Tyr Ala Ser Trp

20                  25                  30

Tyr Gln Gln Lys Pro Gly Gln Ala Pro Val Leu Val Ile Tyr Gly Lys
        35                  40                  45

Asn Asn Arg Pro Ser Gly Ile Pro Asp Arg Phe Ser Gly Ser Ser Ser
    50                  55                  60

Gly Asn Thr Ala Ser Leu Thr Ile Thr Gly Ala Gln Ala Glu Asp Glu
65                  70                  75                  80

Ala Asp Tyr Tyr Cys Asn Ser Arg Asp Ser Ser Gly Asn His Val Val
                85                  90                  95

Phe Gly Gly Gly Thr Lys Leu Thr Val Leu
            100                 105

<210>  3
<211>  116
<212>  PRT
<213>  Homo sapiens

<400>  3

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Gly Arg Ile Lys Ser Lys Thr Asp Gly Gly Thr Thr Asp Tyr Ala Ala
    50                  55                  60

Pro Val Lys Gly Arg Phe Thr Ile Ser Arg Asp Asp Ser Lys Asn Thr
65                  70                  75                  80

Leu Tyr Leu Gln Met Asn Ser Leu Lys Thr Glu Asp Thr Ala Val Tyr
                85                  90                  95

Tyr Cys Ala Arg Gln Lys Arg Lys Lys Asp Ser Trp Gly Gln Gly Thr
            100                 105                 110

Leu Val Thr Val
        115

<210>  4

<211>  117
<212>  PRT
<213>  Homo sapiens

<400>  4

Met Ala Glu Val Gln Leu Val Glu Ser Gly Gly Gly Leu Val Gln Pro
1               5                   10                  15

Gly Gly Ser Leu Arg Leu Ser Cys Ala Ala Ser Gly Phe Thr Phe Ser
            20                  25                  30

Ser Tyr Trp Met His Trp Val Arg Gln Ala Pro Gly Lys Gly Leu Val
        35                  40                  45

Trp Val Ser Arg Ile Asn Ser Asp Gly Ser Ser Thr Ser Tyr Ala Asp
    50                  55                  60

Ser Val Lys Gly Arg Phe Thr Ile Ser Arg Asp Asn Ala Lys Asn Thr
65                  70                  75                  80

Leu Tyr Leu Gln Met Asn Ser Leu Arg Ala Glu Asp Thr Ala Val Tyr
                85                  90                  95

Tyr Cys Ala Arg Arg Arg Pro Thr Gly His Trp His Trp Gly Gln Gly
            100                 105                 110

Thr Leu Val Thr Val
        115

<210>  5
<211>  355
<212>  DNA
<213>  Homo sapiens

<400>  5
ggcccaggtg cagctggtgc agtctggggc tgaggtgaag aagcctgggg ctacagtgaa      60

aatctcctgc aaggtttctg gatacacctt caccgactac tacatgcact gggtgcaaca     120

ggcccctgga aaagggcttg agtggatggg acttgttgat cctgaagatg gtgaaacaat     180

atacgcagag aagttccagg gcagagtcac cataaccgcg gacacgtcta cagacacagc     240

ctacatggag ctgagcagcc tgagatctga ggacacggcc gtgtattact gtgcaagagg     300

ggctaaggcg actctgggtt tgtggggcca aggtaccctg gtcaccgtgt cgaga          355

<210>  6
<211>  321
<212>  DNA
<213>  Homo sapiens

<400>  6

ctgagctgac tcaggaccct gctgtgtctg tggccttggg acagacagtc aggatcacat 60

gccaaggaga cagcctcaga agctattatg caagctggta ccagcagaag ccaggacagg 120

cccctgtact tgtcatctat ggtaaaaaca accggccctc agggatccca gaccgattct 180

ctggctccag ctcaggaaac acagcttcct tgaccatcac tggggctcag gcggaagatg 240

aggctgacta ttactgtaac tcccgggaca gcagtggtaa ccatgtggta ttcggcggag 300

ggaccaagct gaccgtccta g 321

<210> 7
<211> 348
<212> DNA
<213> Homo sapiens

<400> 7
gaggtgcagc tggtggagtc tgggggaggc ttggtaaagc ctggggggtc ccttagactc 60

tcctgtgcag cctctggatt cactttcagt aacgcctgga tgagctgggt ccgccaggct 120

ccagggaagg ggctggagtg ggttggccgt attaaaagca aaactgatgg tgggacaaca 180

gactacgctg cacccgtgaa aggcagattc accatctcaa gagatgattc aaaaaacacg 240

ctgtatctgc aaatgaacag cctgaaaacc gaggacacgg ccgtgtatta ctgtgcaagg 300

cagaagcgga agaaggattc ttggggccaa ggtaccctgg tcaccgtg 348

<210> 8
<211> 571
<212> DNA
<213> Homo sapiens

<400> 8
catggccgag gtgcagctgg tggagtctgg gggaggctta gttcagcctg gggggtccct 60

gagactctcc tgtgcagcct ctggattcac cttcagtagc tactggatgc actgggtccg 120

ccaagctcca gggaaggggc tggtgtgggt ctcacgtatt aatagtgatg ggagtagcac 180

aagctacgcg gactccgtga agggccgatt caccatctcc agagacaacg ccaagaacac 240

gctgtatctg caaatgaaca gtctgagagc cgaggacacg gccgtgtatt actgtgcaag 300

acgtaggcct acgggtcatt ggcattgggg ccaaggtacc ctggtcaccg tgtcgagagg 360

tggaggcggt tcaggcggag gtggctctgg cggtggcgga tcgtctgagc tgactcagga 420

ccctgctgtg tctgtggcct tgggacagac agtcaggatc acatgccaag gagacagcct 480

cagaagctat tatgcaagct ggtaccagca gaagccagga caggcccctg tacttgtcat 540

ctatggtaaa aacaaccggc cctcagggat c 571

<210> 9
<211> 20
<212> DNA
<213> Artificial Sequence

<220>
<223>  Synthetic oligonucleotide

<400>  9
gaccccaaaa tcagcgaaat                                                    20


<210>  10
<211>  24
<212>  DNA
<213>  Artificial Sequence

<220>
<223>  synthetic oligonucleotide

<400>  10
tctggttact gccagttgaa tctg                                               24


<210>  11
<211>  20
<212>  DNA
<213>  Artificial Sequence

<220>
<223>  synthetic oligonucleotide

<400>  11
gccactcacc tcttcagaac                                                    20


<210>  12
<211>  20
<212>  DNA
<213>  Artificial Sequence

<220>
<223>  synthetic oligonucleotide

<400>  12
agcatccatc tttttcagcc                                                    20


<210>  13
<211>  21
<212>  DNA
<213>  Artificial Sequence

<220>
<223>  synthetic oligonucleotide

<400>  13
cctgcctaac atgcttcgag a                                                  21


<210>  14
<211>  22
<212>  DNA
<213>  Artificial Sequence

<220>
<223>  synthetic oligonucleotide

<400>  14
tgtccagctg atccttcatt tg                                                22

<210>  15
<211>  20
<212>  DNA
<213>  Artificial Sequence

<220>
<223>  synthetic oligonucleotide

<400>  15
tgatggccct gtgccttagt                                                   20

<210>  16
<211>  21
<212>  DNA
<213>  Artificial Sequence

<220>
<223>  synthetic oligonucleotide

<400>  16
ggatccatca gaagctttgc a                                                 21

<210>  17
<211>  24
<212>  DNA
<213>  Artificial Sequence

<220>
<223>  synthetic oligonucleotide

<400>  17
aggcattttg aagaattgga aaga                                              24

<210>  18
<211>  24
<212>  DNA
<213>  Artificial Sequence

<220>
<223>  synthetic oligonucleotide

<400>  18
agtaaaagga gacaatttgg ctct                                              24

<210>  19
<211>  21
<212>  DNA
<213>  Artificial Sequence

<220>
<223>  synthetic oligonucleotide

<400>  19
tctctctaat cagccctctg g                                                 21

<210>  20
<211>  18
<212>  DNA
<213>  Artificial Sequence

<220>
<223>  synthetic oligonucleotide

<400>  20
gctacatggg ctacaggc                                                    18